# EUROPEAN PATENT APPLICATION

(11) **EP 3 854 372 A1**
(43) Date of publication of application: **28.07.2021**
(21) Application number: 21152658.7
(22) Date of filing: 21.01.2021
(51) Int. Cl.: A61J 7/00, A61J 7/04, G16H 20/13

(54) **DOSING DEVICE**

(30) Priority: 21.01.2020 DK PA202000069
(71) Applicant: DoseSystem A/S, 1150 København K (DK)
(72) Inventor: Kofoed Thomsen, Jesper, 3060 Espergærde (DK)
(74) Representative: Andreasen, Søren Laursen Vasegaard

(57) **Abstract**

A non-motorised dosing device (2) comprising a housing (4) having a space (34) configured to receive and contain medication (30). The dosing device (2) comprises an outlet (12) for dispensing the medication (30). The dosing device (2) comprises a manually driven drive assembly configured to move a prepacked package of medication (30), preferably tablets (30), from a first position within the housing (4) to a second position outside the housing (4) upon being manually activated.

## Description

### Field of invention

The present invention relates to a medicine reminder and dosing device for reminding a user to take a predefined medication at a prescribed time.

### Prior art

The prior art has presented various devices configured to provide timely medication of patients. These devices are designed to remind patients to take their medicines, in particular patients who are required to follow a complicated scheme of administration with several doses a day and/or of different medications as well.

The prior art discloses various motorized dosing devices for dosing e.g. tablets from blister packs to a patient, in which these dosing devices have a programmable control unit for controlling the operation of the dosing device. The prior art also comprises solutions in which the user has to pull the package manually. One drawback associated with this solution is, however, the risk of pulling too much in the package so that more packages than required are pulled out. This may pose a serious challenge to the patient as packages that have been pulled out cannot be inserted into the dosing device in an easy manner. If too many packages are cut, the patient cannot use the dosing device to perform the next dosing.

Thus, there is a need for a method and an apparatus that reduce or even eliminate the above-mentioned disadvantages of the prior art.

The prior art solutions are expensive and complex. Accordingly, it is an object of the present invention to provide a dosing device that is less complex and less expensive than the prior art solutions. It is also an object to provide a dosing device that does not require the user to pull the package manually. It is a further object to provide a dosing device which reduce or eliminate the risk of releasing too many packages.

In the following, the "medicine reminder and dosing device" according to the invention is referred to as a "dosing device".

### Summary of the invention

The object of the present invention can be achieved by a dosing device as defined in claim 1 and by a system having the features as defined in claim 13. Preferred embodiments are defined in the dependent subclaims, explained in the following description and illustrated in the accompanying drawings.

The dosing device according to the invention is a non-motorised dosing device comprising a housing with a space configured to receive and contain medication. The dosing device comprises an outlet for dispensing the medication, wherein the dosing device comprises a manually driven drive assembly configured to move a prepacked package of medication, preferably medication in the form of tablets, from a first position within the housing to a second position outside the housing upon being manually activated. The dosing device also comprises an identifier device and a trigger device, wherein the trigger device is arranged and configured to detect when the prepacked package of medication has been moved in a predefined manner from the first position towards the second position. The trigger device is arranged and configured to activate the identifier device when the trigger device has been activated, wherein the identifier device is arranged and configured to identify the identity of the prepacked package.

Hereby, it is possible to provide a dosing device that does not require the user to pull the package manually and that reduces or even eliminates the risk of releasing too many packages. Moreover, the dosing device will automatically identify the identity of the prepacked package in a reliable manner. The dosing device also enables power saving as the identifier device is only activated when identification of a prepacked package is required.

In one embodiment, the dosing device comprises a manually driven drive assembly configured to move a prepacked package of medication, preferably medication in the form of tablets, from a position outside the housing to a position within the housing upon being manually activated. Hereby, it is possible to guide one or more packages into the housing if too many packages have been brought outside the housing. Hereby, the user can still use the dosing device to dose the next package.

Since the dosing device comprises no motor, the cost can be reduced.

In one embodiment, the trigger device comprises a motion sensor that is arranged and configured to detect when the prepacked package of medication has been moved in the predefined manner.

In one embodiment, the motion sensor is an accelerometer arranged and configured to detect the acceleration of the prepacked package of medication or a structure of the manually driven drive assembly, wherein the dosing device is configured to determine if the detected acceleration exceeds a predefined level over a predefined period of time. In one embodiment, the period of time is 500 milliseconds or more.

In one embodiment, the motion sensor is a gyroscope arranged and configured to detect the orientation and angular velocity of the prepacked package of medication or a structure of the manually driven drive assembly, wherein the dosing device is configured to determine if the detected orientation and angular velocity are no longer within a predefined time range. In one embodiment, the period of time is 500 milliseconds or more.

In one embodiment, the dosing device comprises an activator arranged to be activated by a user, wherein the dosing device is configured to:
a) initiate that information is sent wirelessly to a recipient or
b) initiate that the dosing device is linked to a smartphone
when the activator is activated (e.g. pressed).

Hereby, it is possible to provide a dosing device that is less complex and less expensive than the prior art solutions.

The trigger device is configured to be triggered when the prepacked package of medication is moved. This may be done by applying a trigger device that detects the motion of the prepacked package of medication. This may alternatively be done by applying a trigger device that detects the motion of a structure which is moved when the prepacked package of medication is moved.

In one embodiment, the trigger device is arranged and configured to activate the identifier device when the trigger device has been activated, wherein the identifier device is arranged and configured to identify the identity of the prepacked package.

In one embodiment, the trigger device is arranged and configured to detect motion of a rotatably mounted knob constituting part of the manually driven drive assembly.

In one embodiment, the trigger device is arranged and configured to detect rotational motion of a shaft that is connected to a rotatably mounted knob constituting part of the manually driven drive assembly.

In one embodiment, the trigger device is arranged and configured to detect linear motion of the prepacked package.

The identifier device is arranged and configured to identify the identity of the prepacked package.

In one embodiment, the trigger device comprises a magnetic field sensor and a permanent magnet, wherein the magnetic field sensor is arranged and configured to detect motion of the permanent magnet, wherein the permanent magnet is attached to a structure that is connected to the manually driven drive assembly. Hereby, a simple, reliable and cost-effective solution can be provided.

In one embodiment, the identifier device is a barcode reader. This embodiment enables reading of barcodes provided on prepacked packages dispensed by the dosing device according to the invention.

In one embodiment, the identifier device is a visual scanner configured to scan a code provided on prepacked packages. This embodiment enables reading of codes provided on prepacked packages dispensed by the dosing device according to the invention. In one embodiment, the code is a Quick Response (QR) code.

In one embodiment, the identifier device comprises a weighing device. Hereby, it is possible to weigh the prepacked packages dispensed by the dosing device in order to identify the packages. This identification step may be combined with one or more additional identification steps such as taking a picture or scanning a code provided on the prepacked packages dispensed by the dosing device.

In one embodiment, the identifier device comprises a camera. Hereby, it is possible to take a picture of the prepacked packages dispensed by the dosing device in order to identify the packages. This identification step may be combined with one or more additional identification steps.

In one embodiment, the dosing device comprises no built-in mobile communication modem configured to apply a global system for mobile communication such as GSM, LTE, IOT, CAT-M1 or any other cellular standard.

In one embodiment, the dosing device comprises a built-in mobile communication modem configured to apply a global system for mobile communication such as GSM, LTE, IOT, CAT-M1 or any other cellular standard.

The medication may be tablets e.g. a prepacked package of tablets. The medication may be a liquid, a gel or a cream.

The wirelessly sent information may be sent via any suitable communication platform. In one embodiment, the communication platform is a wireless network. In one embodiment, the communication platform is a Low Power Wide Area wireless network.

In one embodiment, the dosing device comprises a narrow band modem. In one embodiment, the narrow band modem is configured to generate modulated output signal having an essential frequency spectrum limited to an extent which can be wholly contained within, and faithfully transmitted through, a voice channel with a nominal 4 kHz bandwidth.

In one embodiment, the dosing device itself does not comprise a communication device. In one embodiment, the dosing device comprises a connection for connecting the dosing device to an external modem. In one embodiment, the dosing device comprises a connection for connecting the dosing device to an external modem via a universal serial bus (USB) interface which enables communication between the dosing device and the external modem.

The activator may be formed as a button arranged to be activated by a user. In a preferred embodiment, the dosing device comprises a control unit electrically connected to the activator through a wired connection, wherein the control unit is configured to initiate that information is sent wirelessly to a recipient when the activator is activated (e.g. pressed). In one embodiment, the recipient forwards information to a smartphone or another device (e.g. PC, tablet computer or cell phone).

In one embodiment, the initiate that the dosing device is linked to a smartphone when the activator is activated (e.g. pressed). Hereby, it is possible to update the firmware of the dosing device.

In one embodiment, the recipient is a cloud-based recipient. In one embodiment, the recipient is an application, service or resource made available to the user on demand via the Internet from a cloud computing provider's server. The cloud computing may be any on-demand available computer system resource providing data storage and computing power.

In one embodiment, the dosing device comprises a control unit electrically connected to the activator through a wired connection, wherein the control unit is configured to initiate that information is sent wirelessly to a recipient when the activator is activated (e.g. pressed).

In one embodiment, the recipient is a smartphone. In one embodiment, the recipient is a cloud-based recipient. In one embodiment, the recipient is an application, service or resources made available to the user on demand via the Internet from a cloud computing provider's server. The cloud computing may be any on-demand available computer system resource providing data storage and computing power.

In one embodiment, the dosing device is configured to communicate wirelessly and receive software update information upon pressing the activator.

In one embodiment, the manually driven drive assembly comprises a first roller arranged to drive a second roller, wherein the rollers are arranged to receive a prepacked package of medication (e.g. tablets) and move the package of medication towards an opening provided in the housing when the first roller is rotated in a first rotational direction.

In a preferred embodiment, the rollers are arranged to move the package of medication in the opposite direction when the first roller is rotated in the opposite rotational direction.

In one embodiment, the manually driven drive assembly comprises a rotatably mounted knob protruding from the housing and being mechanically connected to a first roller arranged to drive a second roller.

Hereby, it is possible to provide a simple an efficient way of manually moving the prepacked package of tablets.

In one embodiment, the rotatably mounted knob protrudes from a side portion of the housing. In one embodiment, the rotatably mounted knob protrudes from an upright side portion of the housing. This may be advantageous since this position enables the user to grab and rotate the knob.

In one embodiment, at least one of the rollers comprises an outer portion made of a resilient material so that the outer portion (the portion of the roller arranged along the radial periphery) of the roller has the capability to absorb energy when it is deformed elastically and release that energy upon unloading.

In one embodiment, both rollers comprise an outer portion (the portion of the roller arranged along the radial periphery) made of a resilient material so that the outer portion has the capability to absorb energy when it is deformed elastically and release that energy upon unloading. Hereby, the rollers can be deformed elastically when a prepacked package of medication (e.g. tablets) is moved by the rollers both pressing against the prepacked package of medication.

In one embodiment, at least the outer portion of one of the rollers is made of resilient silicone rubber.
In one embodiment, at least the outer portion of one of the rollers is made of natural rubber.

In one embodiment, at least the outer portion of one of the rollers is made of foam rubber.

It may be an advantage that the housing is configured to receive a prepacked package of tablets, wherein the package comprises a plurality of containers each comprising a number of tablets, wherein the package of tablets comprises machine-readable information about the tablets, wherein the dosing device is configured to allow the prepacked package of tablets to be rotated when the prepacked package of tablets is not surrounding any shaft in the housing. Hereby, the insertion of the prepacked package of tablets is easier and faster than when applying the prior art dosing devices.

It may be advantageous that the dosing device comprises a control unit and one or more ports, wherein each of the one or more ports is configured to electrically connect an external device to the control unit.

Hereby, it is possible to electrically connect one or more external devices. Accordingly, it is possible to provide a simple dosing device which can be equipped and hereby upgraded by connecting one or more external devices without increasing the complexity of the dosing device.

By way of example, the external device may be a tracker comprising an internal satellite-based navigation device configured to determine the location of the dosing device. The satellite-based navigation device may be a GPS receiver, a GLONASS navigation device or a Galileo navigation device.

By way of example, the external device may be a transmitter comprising a mobile communication modem, which may be configured to apply a global system for mobile communication such as GSM, LTE, IOT, CAT-M1 or any other cellular standard. It is possible to apply any desired operating frequency band. Accordingly, the mobile communication modem may be operated at any of the following frequency bands: 850MHz, 900MHz, 1800MHz and 1900MHz. It is important to underline that the mobile communication modem may be operated at any suitable frequency band.

By way of example, the external device is a temperature logger configured to detect and store temperature data.

In one embodiment, the one or the one or more ports is/are an Universal Serial Bus port.

In one embodiment, the dosing device comprises a slidably arranged button, wherein the button is arranged to be pressed towards the bottom of the housing. Hereby, it is possible to manually activate the button in order to initiate any desired manual operation.

In one embodiment, the cutter is arranged to be manually activated and manually driven. Hereby, it is possible to provide a reliable and simple dosing device.

It may be beneficial that the dosing device comprises a cutter arranged to cut prepacked packages of tablets, wherein the cutter is slidably arranged in the housing and mechanically connected to the slidably arranged button. Hereby, it is possible to provide a simple, reliable and user-friendly dosing device.

In a preferred embodiment, the dosing device comprises a scanner arranged and configured to scan machine-readable information about the tablets. Hereby, it is possible to carry out an automatic identification of the machine-readable information. In one embodiment, the dosing device comprises a control unit configured to make sure that an acknowledgement is sent to one or more external receivers.
In a preferred embodiment, the scanner is arranged and configured to scan a one-dimensional barcode or a two-dimensional barcode (RQ code).

In one embodiment, the housing comprises a rear portion and a front portion, wherein the rear portion is detachably attached to the front portion. Hereby, the user can detach the rear portion from the front portion and use the rear portion without the front portion.

In one embodiment, the dosing device comprises two mechanical attachment structures configured to mechanically attach the rear portion to the front portion. Hereby, it is possible to lock the rear portion to the front portion.

In one embodiment, the mechanical attachment structures are formed as two eyes. Hereby, the rear portion can be locked to the front portion by using a padlock. Alternatively, the rear portion and the front portion can be sealed.

In one embodiment, the button is protruding upwards from the top portion of the housing. Hereby, access to the button is improved.

In one embodiment, a display is provided as an integrated part of the button.

In one embodiment, the button extends along more than half of the width of the housing.

In one embodiment, the button extends along more than two-thirds of the width of the housing.

In one embodiment, the button extends along more than three-fourths of the width of the housing. Hereby, it is easier to press down the button.
In one embodiment, the dosing device comprises a motion sensor arranged and configured to detect the motion of a package of prepacked medication.

In one embodiment, the motion sensor is an accelerometer.

It may be an advantage that the dosing device is configured to start-up the scanner automatically when the motion sensor has detected a motion of the package of prepacked medication.

In one embodiment, the dosing device comprises a verification device arranged and configured to detect the content of the prepacked package of medication and verify that the content matches the expected content. In one embodiment, the verification device is configured to detect a code on the prepacked package and identify one or more parameters of the medication. In one embodiment, the verification device is configured to detect the number of pills in the prepacked package. In one embodiment, the verification device is configured to detect the weight of the pills in the prepacked package. In one embodiment, the verification device is configured to detect the appearance (e.g. colour and/or size) of the pills in the prepacked package.

In one embodiment, the dosing device comprises a communication unit by means of which it is possible to receive information from a user and/or send information to a user. This may be an advantage during a test scenario.

The system of the invention is a system comprising a dosing device according to the invention, wherein the system comprises an additional dosing device configured to communicate wirelessly with a smartphone, wherein the smartphone is used to scan the barcode of:
a) a package of medication or
b) a predefined side medication (not from the package)
and wirelessly send notification information to an external receiver (e.g. a server),
wherein the additional dosing device comprises an acknowledgement button which is an integrated part of the additional dosing device and which is configured to be presses when a user has taken a predefined side medication that does not originate from the package, wherein the additional dosing device is configured to communicate via any suitable communication platform. In one embodiment, the communication platform is a wireless network.

### Description of the Drawings

The invention will become more fully understood from the detailed description given herein below. The accompanying drawings are given by way of illustration only, and thus, they are not limitative of the present invention. In the accompanying drawings:
- Fig. 1A: shows a perspective top view of a schematic side view of a dosing device according to the invention;
- Fig. 1B: shows a first side view of the dosing device shown in Fig. 1A;
- Fig. 2A: shows a top view of the dosing device shown in Fig. 1A;
- Fig. 2B: shows a second side view of the dosing device shown in Fig. 1A;
- Fig. 2C: shows a front view of the dosing device shown in Fig. 1A;
- Fig. 3A: shows a first cross-sectional view of the dosing device shown in Fig. 1A;
- Fig. 3B: shows a second cross-sectional view of the dosing device shown in Fig. 1A;
- Fig. 4A: shows a top view of a dosing device according to the invention in a configuration in which the lid has been removed;
- Fig. 4B: shows a perspective top view of the dosing device shown in Fig. 4A;
- Fig. 4C: shows a perspective bottom view of the dosing device shown in Fig. 4A;
- Fig. 4D: shows a side view of the dosing device shown in Fig. 4A;
- Fig. 5A: shows a cross-sectional view of the dosing device shown in Fig. 1A in a configuration in which a prepacked package of tablets is arranged in the housing of the dosing device;
- Fig. 5B: shows a shaft with a permanent magnet, wherein the shaft is arranged in a first configuration;
- Fig. 5C: shows the shaft shown in Fig. 5B, wherein the shaft is arranged in a second configuration;
- Fig. 5D: shows the shaft shown in Fig. 5B, wherein the shaft is arranged in a second configuration;
- Fig. 6A: shows an overview of a dosing system according to the invention;
- Fig. 6B: shows a schematic view of a barcode reader that reads a barcode of a prepacked package of tablets dispensed by a dosing device according to the invention;
- Fig. 7: shows an overview of a different dosing solution according to the invention;
- Fig. 8: shows another overview of a different dosing solution according to the invention;
- Fig. 9A: shows an end view of a dosing device according to the invention;
- Fig. 9B: shows a side view of the dosing device shown in Fig. 9A;

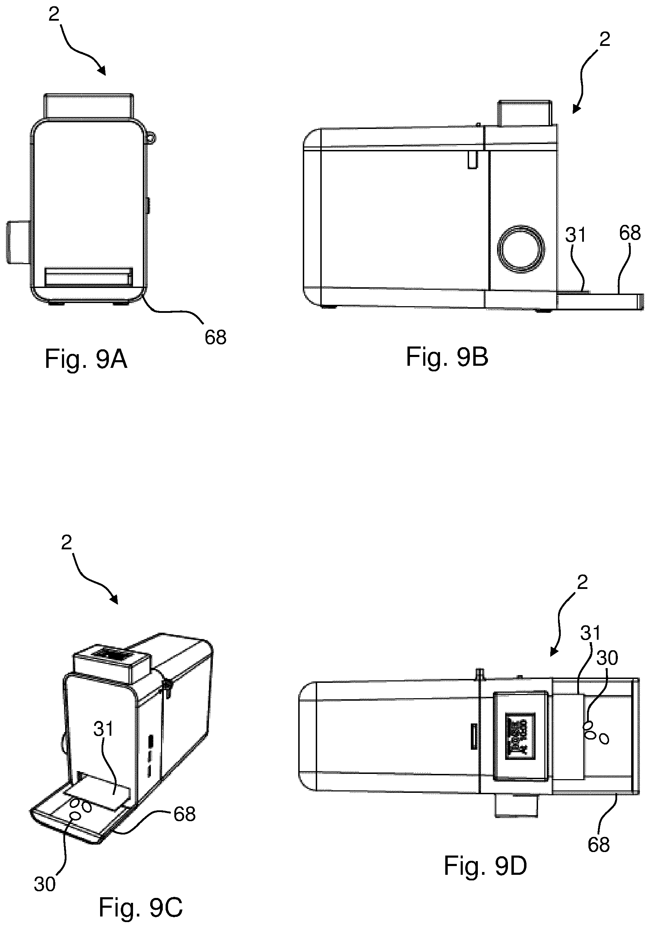

- Fig. 9A: shows a perspective top view of the dosing device shown in Fig. 9A;
- Fig. 9D: shows a top view of the dosing device shown in Fig. 9A;

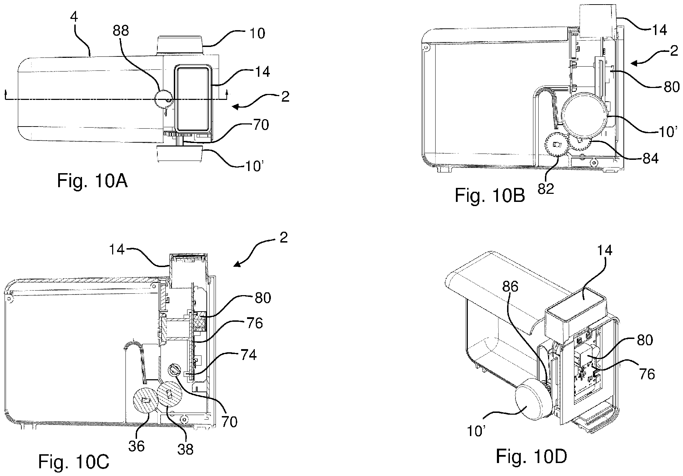

- Fig. 10A: shows a top view of a dosing device according to the invention;
- Fig. 10B: shows a first side view of the dosing device shown in Fig. 10A;
- Fig. 10C: shows a second side view of the dosing device shown in Fig. 10A;
- Fig. 10D: shows a perspective side view of the dosing device shown in Fig. 10B;

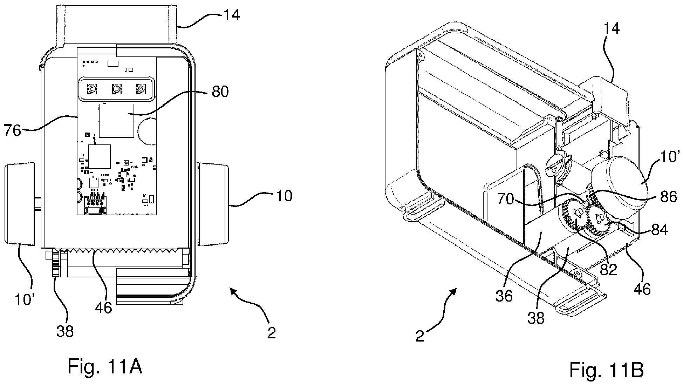

- Fig. 11A: shows an end view of the dosing device shown in Fig. 10A and
- Fig. 11B: shows a perspective bottom view of the dosing device shown in Fig. 10B.

### Detailed description of the invention

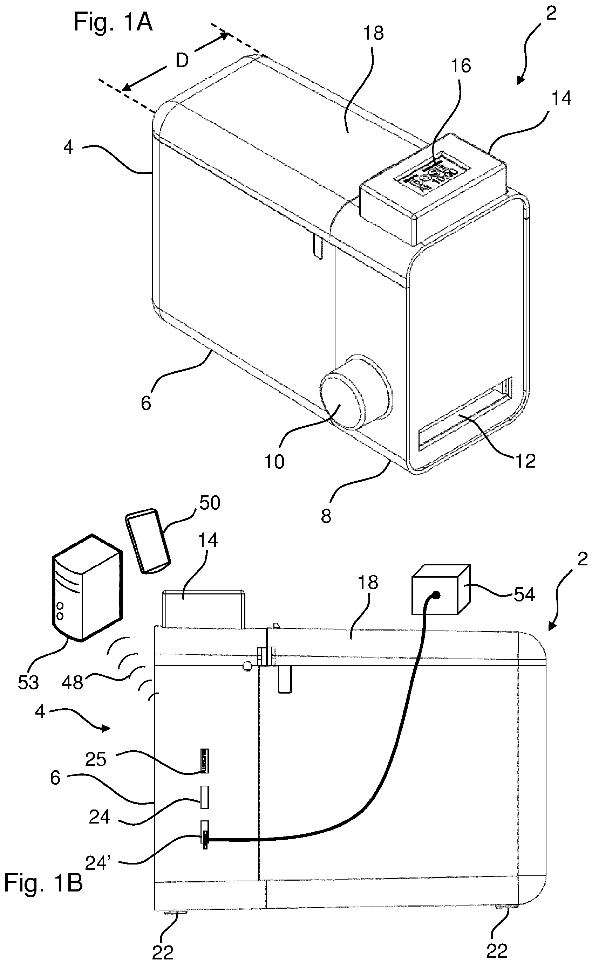

Referring now in detail to the drawings for the purpose of illustrating preferred embodiments of the present invention, a dosing device 2 of the present invention is illustrated in Fig. 1A.

Fig. 1A illustrates a perspective top view of a schematic side view of a dosing device 2 according to the invention. The dosing device 2 comprises a housing 4 having a space configured to receive and contain medication such as a prepacked package of tablets, wherein the package comprises a plurality of containers each comprising a number of tablets. Such packages are well-known and will typically comprise machine-readable information (e.g. a machine-readable optical label which contains information about the item to which it is attached). Such a label may comprise a one-dimensional barcode or a two-dimensional barcode, also known as a Quick Response (QR) code.

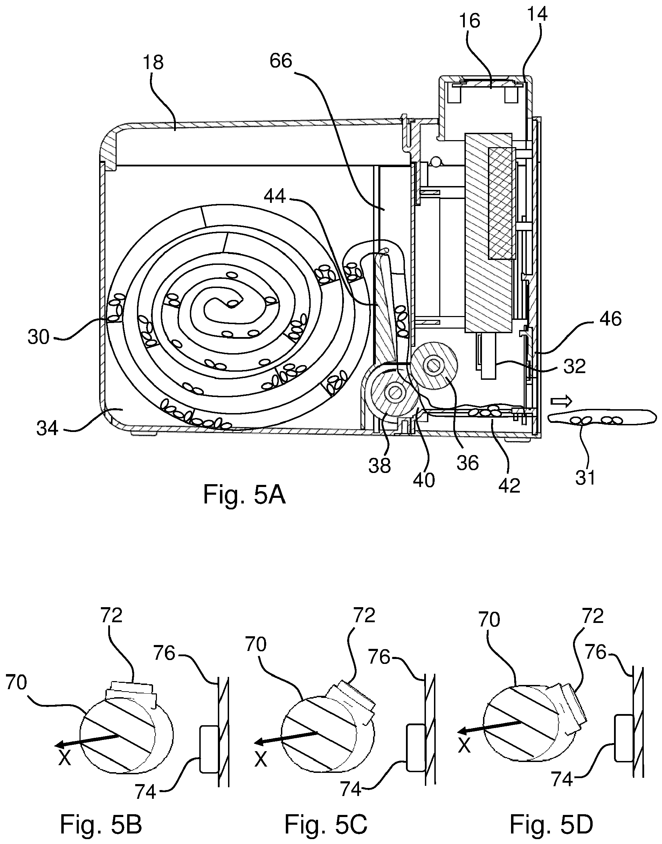

The dosing device 2 is configured to allow the prepacked package of tablets (see Fig. 5A) to be rotated - without arranging the prepacked package of tablets - in a manner in which the package surrounds a shaft in the housing 4.

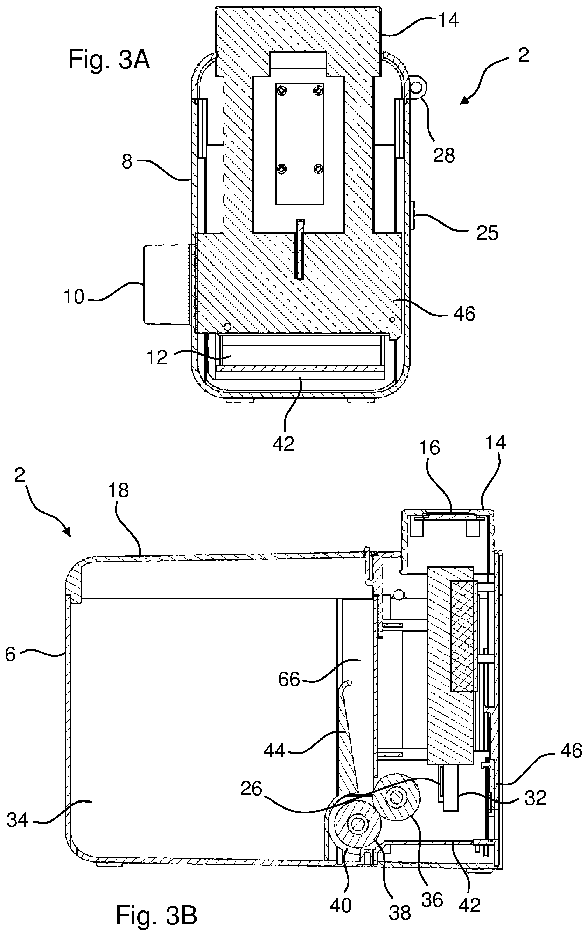

The dosing device 2 comprises a rotatably mounted knob 10 protruding from an upright side portion of the housing 4. As illustrated in Fig. 3B, the knob 10 is mechanically connected to a first roller arranged to drive a second roller of a transportation arrangement adapted for moving packages received by the dosing device 2. The dosing device is non-motorised, and thus the motion of the packages is manually driven (by rotation of the knob).
The dosing device 2 comprises a slidably arranged button 14. The button 14 is arranged to be pressed towards the bottom of the housing 4. In Fig. 1A, the button 14 protrudes from the top portion of the housing 4. A lid 18 constitutes the top portion of the dosing device 2. The button 14 is mechanically connected to a slidably arranged cutter configured to either cut a package transported by the transportation arrangement of the dosing device 2 or fix the package to a support structure, hereby allowing the package to be torn. Hereby, the dosing device 2 is capable of delivering/dosing a single portion of the package through an outlet (opening) 12 provided in the front portion 8 of the housing 4.

A display 16 is provided as an integrated part of the button 14. The button 14 extends along more than two-thirds of the width D of the housing 4.

Fig. 1B illustrates a first side view of the dosing device 2 shown in Fig. 1A. It can be seen that the button 14 protrudes from the top portion of the front portion 8 of the housing 4. The bottom portion of the housing 4 is provided with feet 22. The dosing device 2 comprises an activator 25 arranged to be activated by a user. The activator 25 is formed as a push button. The dosing device 2 comprises a control unit (not shown) electrically connected to the activator 25 and configured to initiate sending information 48 wirelessly to a cloud-based recipient 53 when the activator 25 is activated (e.g. pressed). Hereby, it is possible to reduce the cost of the dosing device 2 significantly compared to existing dosing devices. In one embodiment, the cloud-based recipient is an application, service or resource made available to the user on demand via the Internet from a cloud computing provider's server 53. The cloud computing may be any on-demand available computer system resource providing data storage and computing power.

In one embodiment, the dosing device 2 comprises a control unit (not shown) electrically connected to the activator 25 and configured to initiate sending information 48 wirelessly to a smartphone 50 when the activator 25 is activated (e.g. pressed).

In one preferred embodiment, the dosing device 2 is configured to send the wireless information 48 via a Low Power Wide Area network such as Sigfox. It is important to underline that any suitable communication platform can be applied. In one embodiment, the communication platform is a wireless network. In one embodiment, the communication platform is Sigfox.

The dosing device 2 comprises several ports 24, 24' each configured to electrically connect an external device 54 to the control unit (not shown) by means of a wired connection (a cable 56).

In one embodiment, the external device 54 is a tracker comprising an internal satellite-based navigation device configured to determine the location of the dosing device 2. The satellite-based navigation device may be a GPS receiver, a GLONASS navigation device or a Galileo navigation device.

In one embodiment, the external device 54 is a transmitter comprising a mobile communication modem which may be configured to apply a global system for mobile communication such as GSM, LTE, IOT, CAT-M1 or any other cellular standard. It is possible to apply any desired operating frequency band. Accordingly, the mobile communication modem may be operated at any of the following frequency bands: 850MHz, 900MHz, 1800MHz and 1900MHz. However, the mobile communication modem may be operated at any other suitable frequency bands.

In one embodiment, the external device 54 is a temperature logger configured to detect and store temperature data.

In one embodiment, one or more of the ports 24, 24' are provided as Universal Serial Bus (USB) ports.

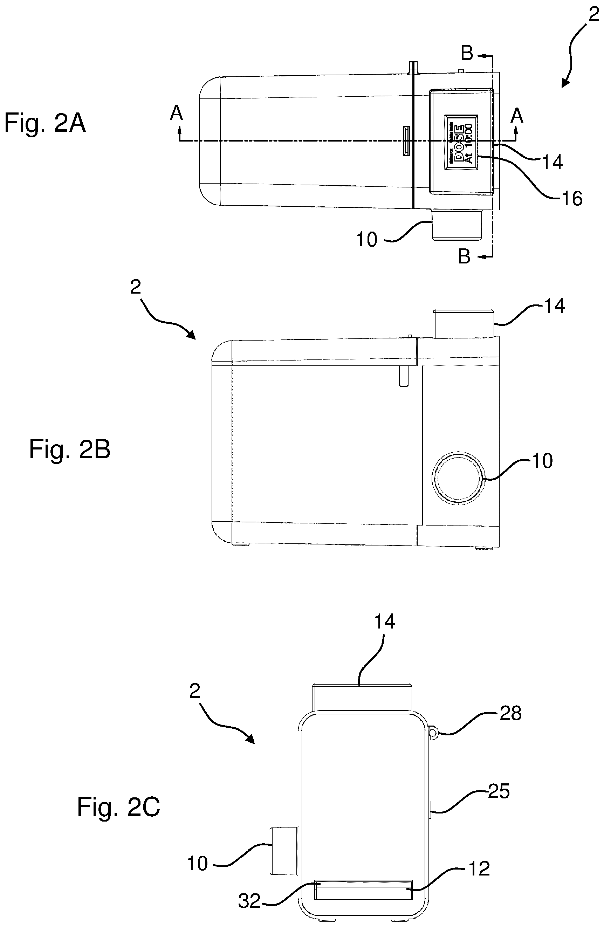

Fig. 2A illustrates a top view of the dosing device 2 shown in Fig. 1A.

Two section lines A, B are illustrated. Corresponding cross-sectional views are shown in Fig. 3A and Fig. 3B.

Fig. 2B illustrates a second side view of the dosing device shown in Fig. 1A.

Fig. 2C illustrates a front view of the dosing device shown in Fig. 1A. It can be seen that the dosing device comprises two mechanical attachment structures 28 configured to mechanically attach the rear portion of the housing to the front portion of the housing.

The mechanical attachment structures 28 are formed as two eyes 28. Accordingly, the rear portion of the housing can be locked to the front portion of the housing by using a padlock or a cable tie. Alternatively, the rear portion of the housing and the front portion of the housing can be sealed by using the attachment structures 28. The dosing device 2 comprises an activator 25 formed as a push button and arranged and configured to be activated by a user.

Fig. 3A illustrates a first cross-sectional view of the dosing device 2 shown in Fig. 1A. The dosing device 2 comprises a button 14 protruding from the top portion of the front portion 8 of the housing. The button 14 is mechanically connected to a cutter 46. The cutter 46 is configured to bear against a prepacked package of medication (e.g. tablets) arranged in the support structure 42 formed as a plate. Hereby, the cutter 46 can tear off one portion of the prepacked package of medication (e.g. tablets) and hereby release the medication to the user through the outlet 12. In one embodiment, the dosing device 2 comprises a protection unit configured to prevent the user from being cut by the cutter 46. In one embodiment, the protection unit comprises a control unit and one or more sensors designed to detect if a hand is in proximity of the cutter 46. In one embodiment, the cutter 46 comprises a safety arrangement configured to prevent the user from being cut by the cutter 46.

A spring member (not shown) is arranged to return the button 14 and thus the cutter 46 to the initial position (as shown in Fig. 3A) when the button 14 is no longer pressed downwards by the user.

The bottom portion of the housing is provided with feet. Furthermore, the dosing device 2 comprises an activator 25 arranged to be formed as a push button. The dosing device 2 comprises a control unit (not shown) that is electrically connected to the activator 25 and configured to initiate sending information wirelessly to a smartphone when the activator 25 is pressed. It can be seen that a knob 10 protrudes from the opposite side of the front portion 8 of the housing than the activator 25.

The dosing device 2 comprises two mechanical attachment structures 28 formed as two eyes 28. One eye 28 is attached to the front portion 8 of the housing and a corresponding eye 28 is attached to the rear portion of the housing. The eyes 28 are arranged in a short distance (preferably less than 5 mm) from each other so that the rear portion of the housing can be locked to the front portion 8 of the housing by using a padlock or a cable tie.

Fig. 3B illustrates a second cross-sectional view of the dosing device shown in Fig. 1A. The housing of the dosing device 2 comprises a rear portion 6 defining a space 34 configured to receive a prepacked package of tablets (as illustrated in Fig. 5A). A lid 18 is detachably attached to the open portion of the housing in order to close the housing. The housing comprises a compartment 66 configured to receive the distal end of a prepacked package of medication (e.g. tablets).

A guiding structure 44 is provided in the compartment 66. The guiding structure 44 guides the prepacked package of medication (see Fig. 5A) towards the bottom portion of the compartment 66 so that the prepacked package of medication can enter between a first rotatably mounted roller 36 and a second rotatably mounted roller 38. The second roller 38 is configured to drive and thus move forward the prepacked package of medication to which the roller 38 is brought in contact with when the prepacked package of medication is arranged between the first rotatably mounted roller 36 and the second rotatably mounted roller 38. The first roller 36 is rotatably mounted in the housing and is arranged to drive the second roller 38 through a mechanical connection (e.g. between the two rollers 36, 38 or alternative through contact structures mechanically connected to the two rollers 36, 38, respectively).

A scanner 32 is arranged to visually detect/scan machine-readable information provided on a prepacked package of medication arranged below the scanner 32. In one embodiment, the machine-readable information is a one-dimensional barcode. In another embodiment, the machine-readable information is a two-dimensional barcode (a RQ code).

The dosing device 2 comprises a sensor 26 arranged and configured to detect motion of a prepacked package of medication below the scanner 32. The dosing device 2 is configured to automatically start up the scanner 32 when motion of a prepacked package of medication below the scanner 32 has been detected by means of the sensor 26. In one embodiment, the sensor 26 is an accelerometer.

The dosing device 2 comprises a button 14 arranged in a resting position, in which it protrudes from the lid 18 of the housing of the dosing device 2. The button 14 is, however, slidably arranged in the housing and configured to be pushed downwards towards the bottom portion of the housing of the dosing device 2, and hereby translate (displace) the cutter 46. Hereby, the user will bring the cutter 46 into contact with the upper side of a prepacked package of medication below cutter 46 in order to tear the package and release the medication (e.g. tablets) to the user through the outlet of the housing (see Fig. 5A).

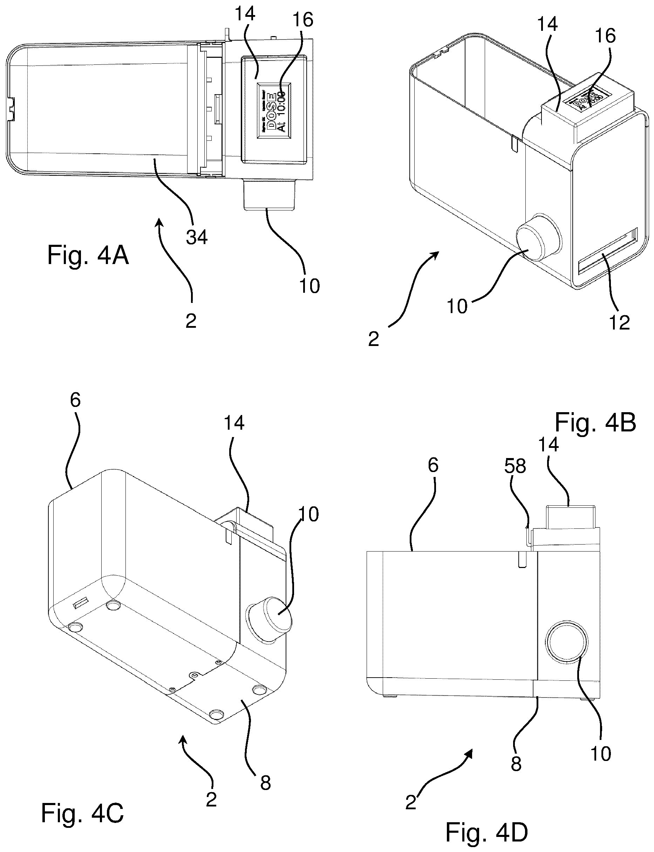

Fig. 4A illustrates a top view of a dosing device 2 according to the invention in a configuration in which the lid has been removed. The dosing device 2 corresponds to the one shown and explained with reference to Fig. 1A, Fig. 1B, Fig. 2A, Fig. 2B, Fig. 2C, Fig. 3A and Fig. 3B. Fig. 4B illustrates a perspective top view of the dosing device 2 shown in Fig. 4A. Fig. 4C illustrates a perspective bottom view of the dosing device 2 shown in Fig. 4A, and Fig. 4D illustrates a side view of the dosing device 2 shown in Fig. 4A.

It can be seen that the dosing device 2 comprises a housing with an attachment structure 58 protruding upwards and configured to receive a corresponding receiving structure of a lid.

Fig. 5A illustrates a cross-sectional view of the dosing device 2 shown in Fig. 1A in a configuration in which a prepacked package of tablets 30 is arranged in the space 34 in the rear portion of the housing of the dosing device 2. It can be seen that the dosing device 2 is configured to receive the prepacked package of tablets 30 without winding the prepacked package of tablets 30 around a shaft. The prepacked package of tablets 30 is simply put into the space 34 and the distal end of the prepacked package of tablets 30 is inserted into the compartment 66 of the housing. The compartment 66 is formed as an elongated space extending along an upright axis of the housing. A guiding structure 44 is arranged in the compartment 66 in order to guide the prepacked package of medication 30 towards the bottom portion of the compartment 66 to facilitate that the prepacked package of medication enters between the first rotatably mounted roller 36 and the second rotatably mounted roller 38.

This roller 38 is configured to drive and thus move forward the prepacked package of medication 30 with which the roller 38 is brought into contact. A first roller 36 is rotatably mounted in the housing and arranged to drive the second roller 38 through a mechanical connection (e.g. between the two rollers 36, 38 or alternative through contact structures mechanically connected to the two rollers 36, 38, respectively).

When the knob (shown in Fig. 4A, Fig. 4B, Fig. 4C and Fig. 4D) is rotated in a clockwise direction, the first roller 36 will rotate in the same direction. Accordingly, the second roller 38 will rotate anticlockwise and hereby force the prepacked package of tablets 30, which is in contact with the second roller 38, to move to the right - towards the outlet of the housing.

When the user can see that the package 31 has been moved out of the outlet, the user can easily press the button 14 downwards to tear off the package 31 from the remaining part of the prepacked package of tablets 30. The cutting process is carried out by letting the distal end of the cutter 46 bear against the upper prepacked package of tablets 30. The cutter 46 may be formed as a knife, a saw or another structure configured to break the prepacked package of tablets 30 or to hold against it, hereby allowing the package 31 to be pulled off the remaining prepacked package of tablets 30 (this is an option if the prepacked package of tablets 30 comprises perforations allowing the package 31 to be pulled off the remaining prepacked package of tablets 30).

Fig. 5B illustrates a shaft 70 with a permanent magnet 72, wherein the shaft 70 is arranged in a first configuration, in which the permanent magnet 72 protrudes basically upwards along the length of a printed circuit board 76. Fig. 5C illustrates the shaft 70 shown in Fig. 5B, wherein the shaft 70 is arranged in a second configuration. Fig. 5D illustrates the shaft 70 shown in Fig. 5B, wherein the shaft 70 is arranged in a second configuration.

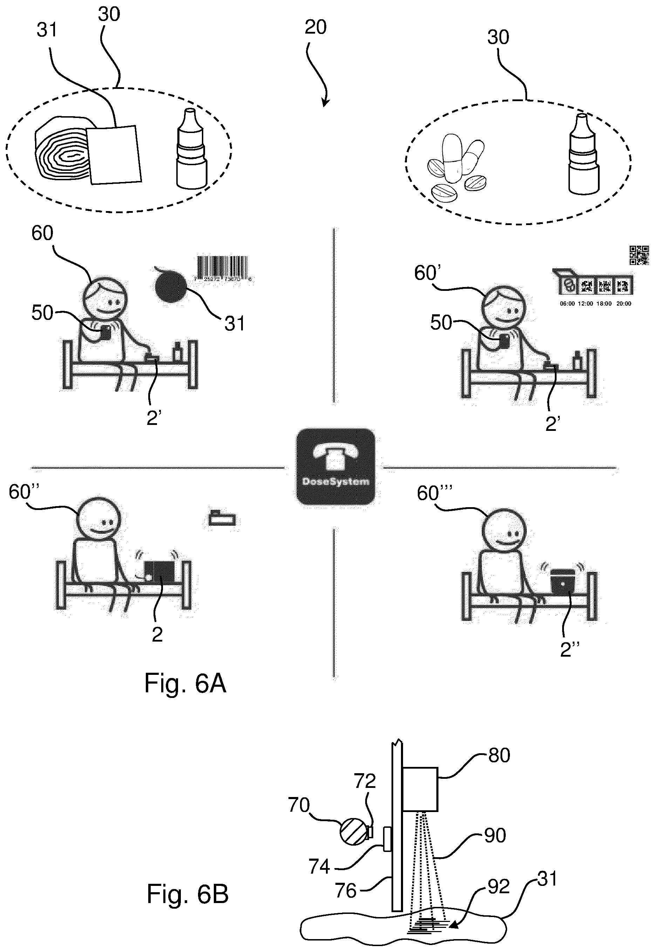

A trigger device 74 formed as a magnetic field sensor 74 is placed on the printed circuit board 76. The magnetic field sensor 74 is arranged and configured to detect rotation of the shaft 70 about its longitudinal axis X. In Fig. 5B and in Fig. 5C, however, the permanent magnet 72 is too far away from the magnetic field sensor 74 to detect the magnetic field of the permanent magnet 72. In Fig. 5D, the shaft 70 has been further rotated into a configuration in which the magnetic field sensor 74 is capable of detecting the magnetic field of the permanent magnet 72. When the magnetic field sensor 74 detects the magnetic field of the permanent magnet 72, the magnetic field sensor 74 activates an identifier device (e.g. a barcode reader or another type of reader such as a QR code reader), which is arranged and configured to identify the prepacked package of tablets provided in a package dispensed by the dosing device (this is schematically illustrated in Fig. 6B).

Fig. 6A illustrates an overview of a dosing system 20 according to the invention. The dosing system 20 is configured to dose a regime of medication 30 in the form of tablets and/or a liquid or gel. The system 20 is configured to dose prepacked package of tablets provided in a package 31.

The dosing system 20 provides a first solution (illustrated in the upper left corner), in which a user 60 applies a smartphone 50 to scan a code (e.g. a barcode) on a package 31 of medication and wirelessly send notification information to an external receiver (e.g. a server). When the user 60 has taken a predefined side medication (not from the package 31), the user 60 presses an acknowledgement button which is an integrated part of a device 2'. The device 2' may comprise a display configured to inform the user 60 about relevant information such as the time to next required dose of medication. The device 2' is configured to communicate by using a suitable communication platform. In one embodiment, the communication platform is a wireless network.
The dosing system 20 provides a second solution (illustrated in the upper right corner), in which a user 60' applies a smartphone 50 to scan a code (e.g. a barcode) associated to a medication and wirelessly send notification information to an external receiver (e.g. a server). When the user 60' has taken a predefined side medication, the user 60' presses an acknowledgement button, which is an integrated part of a device 2'. The device 2' may comprise a display configured to inform the user 60 about relevant information such as the time to next required dose of medication. The device 2' is configured to communicate by using a suitable communication platform. In one embodiment, the communication platform is a wireless network.

The dosing system 20 provides a third solution (illustrated in the lower left corner), in which a user 60" applies a dosing device 2 to scan a package 31 of medication. This dosing device 2 corresponds to the one explained with reference to precious figures (Fig. 1-5).

The dosing system 20 provides a fourth solution (illustrated in the lower right corner), in which a user 60'" applies a dosing device 2" to provide the prescribed medication at the prescribed time.

The system 20 is flexible with respect to these four solutions, in which the first solution is cheapest, and the second solution is intended for young individuals who are familiar with using a smartphone 50. The third solution is also relatively cheap and can be used without a smartphone if another gateway (e.g. a local area network such as a WIFI-connection) or an external GSM module is provided. The fourth solution is intended for older individuals who are less familiar with using a smartphone 50 than young individuals.

Fig. 6B illustrates a schematic view of an identifier device 80 formed as a barcode reader 80, which is configured to read a barcode of a prepacked package 31 of tablets dispensed by a dosing device according to the invention. It is important to note that the identifier device 80 may be formed as another type of reader (e.g. a QR code reader). The dosing device comprises a trigger device 74 as the one shown in and explained with reference to Fig. 5B, Fig. 5C and Fig. 5D.

The trigger device 74 is formed as a magnetic field sensor 74 and is placed on the printed circuit board 76. In Fig. 6B, the shaft 70 is arranged in a configuration in which the magnetic field sensor 74 is capable of detecting the magnetic field of the permanent magnet 72. Accordingly, the magnetic field sensor 74 has detected the magnetic field of the permanent magnet 72. Therefore, the trigger device 74 has activated the identifier device 80 formed as a barcode reader 80. The identifier device 80 may be formed as another type of reader (e.g. a QR code reader). Now the barcode reader 80 identifies the identity of the prepacked package 31 dispensed by the dosing device. This is done by sending light 90 towards the barcode 92 and sensing the reflected signal.

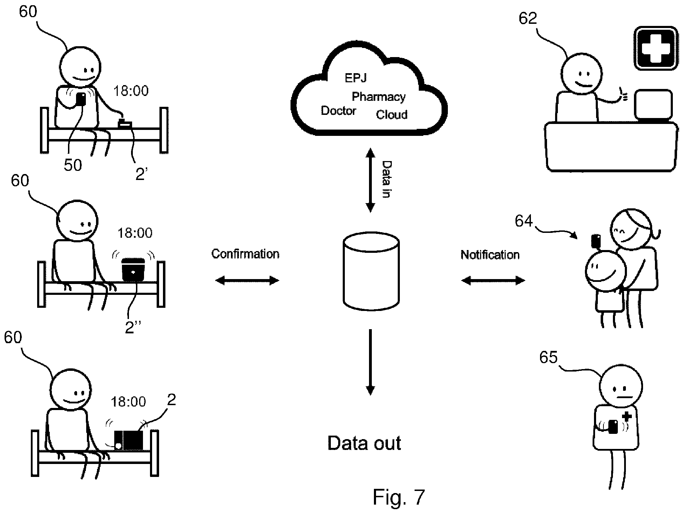

Fig. 7 illustrates an overview of a different dosing solution according to the invention. It can be seen that a user 60 may use various devices 2, 2', 2" to acknowledge that a prescribed medication has been taken. This requires a smartphone 50 if the device 2' shown in the upper left corner is used. However, confirmation is wirelessly sent without a smartphone 50 when the user 60 applies one of the other devices 2, 2". It can be seen that various individuals including a dispensing chemist 62, relatives 64 and a health care professional 65 can be notified when an acknowledgement has or has not been receive in due time.

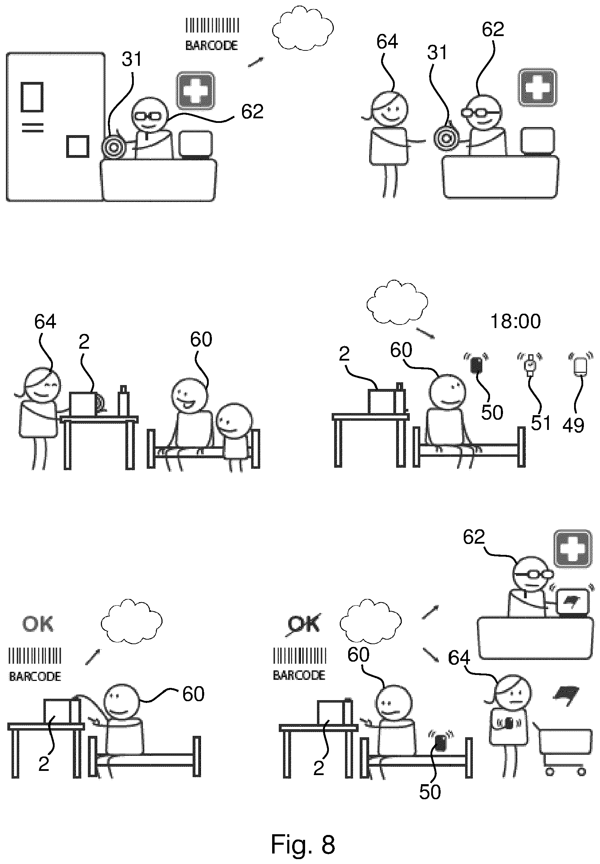

Fig. 8 illustrates another overview of a different dosing solution according to the invention. In the upper left-side corner, a dispensing chemist 62 prepares a package 31 of medication to a user (a patient). A barcode associated with the medication is registered and sent to a cloud-based storage solution. In the upper right-side corner, the relative 64 of a user 60 picks up the package 31 of medication.

In the middle left-side section, the relative 64 helps the user 60 by putting the package 31 of medication into a dosing device 2 according to the invention.

In the middle right-side section, the user 60 receives a reminder by one or more of the following devices: a smartphone 50, a smart watch 51 or a smart speaker 49. Information is sent from the cloud-based solution to the devices 49, 50, 51.

In the lower left-side corner, the user 60 applies a dosing device 2 to acknowledge that the prescribed medication has been taken. Information is sent to a cloud-based solution (e.g. by using a smartphone, a GSM module or another available communication solution).

In the lower right-side corner, the user 60 applies a dosing device 2 to try to acknowledge that the prescribed medication has been taken. However, something goes wrong, and the relative 64 as well as the dispensing chemist 62 are notified by receiving information from the cloud-based solution.

Fig. 9A illustrates an end view of a dosing device 2 according to the invention. The dosing device 2 basically corresponds to the one shown in Fig. 2A, Fig. 2B, Fig. 2C, Fig. 3A and Fig. 3B. However, the dosing device 2 comprises a tray 68 arranged and configured to collect the medication (e.g. pills 30) once a package is cut by the cutter (not shown) of the dosing device 2.

In one embodiment, the tray 68 is detachably attached to the housing of the dosing device 2.

Fig. 9B illustrates a side view of the dosing device 2 shown in Fig. 9A. It can be seen that the dosing device 2 comprises a tray 68 protruding from the housing of the dosing device 2. The tray 68 extends in extension of the housing of the dosing device 2. Furthermore, the tray 68 is arranged to receive medication from a package 31 leaving an opening in the housing.

Fig. 9C illustrates a perspective top view of the dosing device 2 shown in Fig. 9A, whereas Fig. 9D illustrates a top view of the dosing device 2 shown in Fig. 9A. It can be seen that pills 30 from the package 31 (that has been cut) have been collected by the tray 68.

Fig. 10A illustrates a top view of a dosing device 2 according to the invention. The dosing device 2 comprises a housing 4 and a first knob 10 protruding from the right side of the housing 4. The dosing device 2 comprises a second knob 10' protruding from the opposite (left) side of the housing 42. The first knob 10 and the second knob 10' are connected by a shaft 70. The dosing device 2 comprises a locking device 88 that can be accessed from the top portion of the housing 4. In one embodiment, the locking device 88 is configured to be opened by using a magnetic key, wherein the locking device 88 comprises engaging structures that can be brought into and out of engagement by rotation of the magnetic key.

The dosing device 2 comprises a slidably arranged button 14. The button 14 is arranged to be pressed towards the bottom of the housing 4. The button 14 is mechanically connected to a slidably arranged cutter configured to either cut a package transported by the transportation arrangement of the dosing device 2 or fix the package to a support structure, hereby allowing the package to be torn over.

Fig. 10B illustrates a first side view of the dosing device 2 shown in Fig. 10A. The dosing device 2 comprises an identifier device 80 formed as a barcode reader 80 that is arranged and configured to identify the identity of a package 31 dispensed by the dosing device 2 as shown in and explained with reference to Fig. 6B. It is important to note that the identifier device 80 may be formed as another type of reader (e.g. a QR code reader).

The dosing device 2 comprises a first gear 82 that engages with a second gear 84 which engages with a gear arranged on the shaft 70 onto which the second knob 10' is arranged (see Fig. 11B for a better view).

Fig. 10C illustrates a second side view of the dosing device 2 shown in Fig. 10A. In this view, the first gear 82, the second gear 84 and the second knob 10' shown in Fig. 10B have been removed. It can be seen that the identifier device 80 formed as a barcode reader 80 is placed on a printed circuit board 76. Moreover, it can be seen that the dosing device 2 comprises a trigger 74 formed as a magnetic field sensor 74 that is placed on the printed circuit board 76. It is important to note that the identifier device 80 may be formed as another type of reader (e.g. a QR code reader). It can be seen that a first roller 36 is attached to the same shaft as the first gear 82 shown in Fig. 10B. Likewise, a second roller 38 is attached to the same shaft as the second gear 84 shown in Fig. 10B. In Fig. 10C, it is possible to see the shaft 70 to which the second knob 10' shown in Fig. 10B is attached.

Fig. 10D illustrates a perspective side view of the dosing device 2 shown in Fig. 10B. It can be seen that a gear 86 is attached to the knob shaft 70 shown in Fig. 10A and Fig. 10C.

Fig. 11A illustrates an end view of the dosing device 2 shown in Fig. 10A. The cutter 46 comprising a barbed portion is visible.

Fig. 11B illustrates a perspective bottom view of the dosing device 2 shown in Fig. 10B. It can be seen that the first roller 36 and the first gear 82 are mounted on the same shaft. Likewise, it can be seen that the second roller 38 and the second gear 84 are mounted on the same shaft. Moreover, the knob 10' and a third gear 86 are mounted on the same knob shaft 70.

### List of reference numerals

- 2: Dosing device
- 2', 2": Device
- 4: Housing
- 6: Rear portion
- 8: Front portion
- 10: Knob
- 12: Outlet
- 14: Button
- 16: Display
- 18: Lid
- 20: System
- 22: Foot
- 24, 24': Port
- 25: Activator
- 26: Sensor
- 28: Eye
- 30: Medication (e.g. tablets)
- 31: Package
- 32: Scanner
- 34: Space
- 36, 38: Roller
- 40: Gap
- 42: Support structure (e.g. plate)
- 44: Guiding structure
- 46: Cutter
- 48: Information
- 49: Smart speaker
- 50: Smartphone
- 51: Smartwatch
- 52: Control unit
- 53: Server
- 54: External device
- 56: Cable
- 58: Attachment structure
- 60, 60': User
- 60", 60''': User
- 62: Pharmacist
- 64: Relative
- 65: Health care professional
- 66: Compartment
- 68: Tray
- 70: Shaft
- 72: Permanent magnet
- 74: Trigger (e.g. magnetic field sensor)
- 76: Printed circuit board
- 80: Identifier device
- 82, 84, 86: Gear
- 88: Locking device
- 90: Light
- 92: Barcode
- D: Width
- X: Longitudinal axis

## Claims

1. A non-motorised dosing device (2) comprises a housing (4) having a space (34) configured to receive and contain medication (30), wherein the dosing device (2) comprises an outlet (12) for dispensing the medication (30), wherein the dosing device (2) comprises a manually driven drive assembly configured to move a prepacked package of medication (30), preferably tablets (30), from a first position within the housing (4) to a second position outside the housing (4) upon being manually activated, **characterised in that** the dosing device (2) comprises an identifier device (80) and a trigger device (74), wherein the trigger device (74) is arranged and configured to detect when the prepacked package (31) of medication (30) has been moved in a predefined manner from the first position towards the second position, wherein the trigger device (74) is arranged and configured to activate the identifier device (80) when the trigger device (78) has been activated, wherein the identifier device (80) is arranged and configured to identify the identity of the prepacked package (31).

2. A dosing device (2) according to claim 1, **characterised in that** the trigger device (74) comprises a motion sensor that is arranged and configured to detect when the prepacked package (31) of medication (30) has been moved in the predefined manner.

3. A dosing device (2) according to claim 2, **characterised in that** the motion sensor comprises a magnetic field sensor (74) and a permanent magnet (72), wherein the magnetic field sensor (74) is arranged and configured to detect motion of the permanent magnet (72), wherein the permanent magnet (72) is attached to a structure (70) that is connected to the manually driven drive assembly.

4. A dosing device (2) according to one of the preceding claims, **characterised in that** the identifier device (80) is a barcode reader (80) or another visual reader such as a QR code reader.

5. A dosing device (2) according to one of the preceding claims, **characterised in that** the manually driven drive assembly comprises a rotatably mounted knob (10) protruding from the housing (4) and being mechanically connected to a first roller (36) arranged to drive a second roller (38), wherein a gap (40) is arranged next to the second roller (38), wherein the second roller (38) is configured to move a prepacked package of tablets (30) upon being rotated, when the prepacked package of tablets (30) is brought into contact with the second roller (38) and is arranged in the gap (40).

6. A dosing device (2) according to claim 5, **characterised in that** the housing (4) is configured to receive a prepacked package of tablets (30), wherein the package (30) comprises a plurality of containers each comprising a number of tablets (30), wherein the package of tablets (30) comprising machine-readable information about the tablets (30), wherein the dosing device (2) is configured to allow the prepacked package of tablets (30) to be rotated when the prepacked package of tablets (30) is not surrounding any shaft in the housing (4).

7. A dosing device (2) according to one of the preceding claims, **characterised in that** the dosing device (2) comprises a control unit (52) and one or more ports (24, 24'), wherein each of the one or more ports (24, 24') is configured to electrically connect an external device (54) to the control unit (52).

8. A dosing device (2) according to one of the preceding claims, **characterised in that** the dosing device (2) comprises a slidably arranged button (14), wherein the button (14) is arranged to be pressed towards the bottom of the housing (4).

9. A dosing device (2) according to claim 8, **characterised in that** the dosing device (2) comprises a cutter (46) arranged to cut a prepacked package of tablets, wherein the cutter (46) is slidably arranged in the housing (4) and is mechanically connected to the slidably arranged button (14).

10. A dosing device (2) according to one of the preceding claims, **characterised in that** the identifier device (80) comprises a scanner (32) arranged and configured to scan machine-readable information about the tablets (30) or the prepacked package (31).

11. A dosing device (2) according to one of the preceding claims, **characterised in that** the dosing device (2) comprises an activator (25) arranged to be activated by a user, wherein the dosing device (2) is configured to:
a) initiate that information (48) is sent wirelessly to a recipient (50, 53) or
b) initiate that the dosing device (2) is linked to a smartphone (50) when the activator (25) is activated (e.g. pressed).

12. A dosing device (2) according to claim 11, **characterised in that** the housing (4) comprises a rear portion (6) and a front portion (8), wherein the rear portion (6) is detachably attached to the front portion (8), wherein the dosing device (2) comprises two mechanical attachment structures (28) configured to mechanically attach the rear portion (6) to the front portion (8).

13. A system (20) comprising a dosing device (2) according to one of the preceding claims, **characterised in that** the system (20) comprises an additional dosing device (2') configured to communicate wirelessly with a smartphone (50), wherein the smartphone (50) is used to scan a barcode of:
a) a package (31) of medication or
b) a predefined side medication (not from the package 31)
and wirelessly send notification information to an external receiver (e.g. a server),
wherein the additional dosing device (2') comprises an acknowledgement button being an integrated part of the additional dosing device (2') and being configured to be presses when a user (60) has taken a predefined side medication that does not originate from the package (31), wherein the additional dosing device (2') is configured to communicate via a suitable communication platform.
